# FASCICULE DE BREVET EUROPEEN

(11) **EP 1 909 665 B1**
(45) Date de publication et mention de la délivrance du brevet: **30.11.2011**
(21) Numéro de dépôt: 06778892.7
(22) Date de dépôt: 20.07.2006
(51) Int. Cl.: A61B 17/70, A61B 17/80

(54) **IMPLANT RACHIDIEN A MOYEN DE BLOCAGE PIVOTANT POUR UNE TIGE DE LIAISON**
WIRBELSÄULENIMPLANTAT MIT SCHWENKBAREM ARRETIERMITTEL ZUR VERBINDUNG EINER STANGE
SPINAL IMPLANT PROVIDED WITH A PIVOTABLE LOCKING MEANS FOR A CONNECTING ROD

(30) Priorité: 02.08.2005 FR 0508224
(43) Date de publication de la demande: 16.04.2008
(73) Titulaire: Euros, 13600 La Ciotat (FR)
(72) Inventeur: ONIMUS, Michel, F-25240 Gellin (FR); CHATAIGNIER, Hervé, F-25320 Boussieres (FR)
(74) Mandataire: Orsini, Fabienne
(86) Numéro de dépôt international: PCT/FR2006/001774
(87) Numéro de publication internationale: WO 2007/014989

(56) Documents cités:
- DE-A1- 4 433 360
- DE-U1- 9 412 744
- FR-A- 2 722 394
- US-B1- 6 572 622
- US-B1- 6 656 179

## Description

### DOMAINE TECHNIQUE AUQUEL SE RAPPORTE L'INVENTION

La présente invention concerne de manière générale les systèmes d'implants rachidiens thoraco-lombaires antérieurs.

Elle concerne plus particulièrement un implant comprenant un socle pourvu d'un logement adapté à recevoir une tige de liaison s'étendant de part et d'autre du socle, et comportant, d'une part, en regard dudit logement une première ouverture traversante qui débouche du côté de la face avant du socle en retrait du fond dudit logement, et, d'autre part, à son extrémité avant une deuxième ouverture traversante, lesdites première et deuxième ouvertures traversantes présentant des axes convergents pour la mise en place de deux vis de fixation du socle à une vertèbre, ainsi que des moyens de blocage de la tige de liaison dans ledit logement comprenant un capot pivotant sur le socle.

Ce type d'implant assemblé avec une tige de liaison est utilisé pour traiter des arthroses, des fractures vertébrales ou pour corriger des déviations de la colonne vertébrale telles qu'une scoliose ou une cyphose.

En général, le chirurgien fixe par voie antérieure un implant par vertèbre puis place dans l'implant une tige préalablement cintrée en fonction du traitement et de la correction à apporter sur la colonne vertébrale.

### ARRIERE-PLAN TECHNOLOGIQUE

On connaît déjà du document US 6 572 622 un implant rachidien du type précité dans lequel la paroi intérieure de la deuxième ouverture traversante prévue à l'extrémité avant du socle comporte une denture adaptée à coopérer avec la tête de la vis de fixation engagée au travers de ladite deuxième ouverture afin de la bloquer dans ladite deuxième ouverture et éviter ainsi un dévissage intempestif de ladite vis de fixation.

Une telle denture est complexe à réaliser et augmente le coût de fabrication de l'implant.

### OBJET DE L'INVENTION

Par rapport à l'état de la technique précité, l'invention propose un nouvel implant rachidien simple d'utilisation, fiable dans le temps et extractif si nécessaire.

Plus particulièrement, l'invention propose un implant rachidien tel que défini en introduction, dans lequel le capot en position de fermeture sur le socle présente une partie d'extrémité avant qui s'étend au-dessus d'une partie de la deuxième ouverture traversante pour former une butée anti-recul de la vis introduite dans ladite deuxième ouverture traversante..

Ainsi, avantageusement dans l'implant rachidien selon l'invention, le capot présente une double fonction à savoir celles de bloquer la tige de liaison sur le socle et de former une butée anti-recul pour une vis de fixation (la vis antérieure ou avant) du socle sur la vertèbre. Plus particulièrement, dans l'implant rachidien selon l'invention, le capot pivotant permet de positionner aisément la tige de liaison dans le logement du socle et procure un maintien de cette tige tout en autorisant sa rotation et/ou sa translation avant le blocage final. En outre, le capot et la tige de liaison placée dans le logement du socle forment un système de butée anti-recul pour les vis de fixation à la vertèbre.

D'autre caractéristiques non limitatives et avantageuses de l'implant rachidien conforme à l'invention sont les suivantes :
- ledit capot est monté à pivotement autour d'un axe de pivotement qui s'étend sensiblement parallèlement à un axe longitudinal du logement selon lequel est destinée à s'étendre ladite tige de liaison ;
- ledit axe de pivotement est rapporté et serré dans des ouvertures du socle ;
- l'axe de pivotement comporte deux extrémités libres de forme sphérique qui dépassent dudit socle ;
- le capot s'étend globalement transversalement audit logement en le recouvrant et il présente de part et d'autre dudit logement une partie d'extrémité arrière pourvue d'un conduit ouvert à chaque extrémité pour la mise en place d'un axe de pivotement et une partie d'extrémité avant agencée pour coopérer avec des moyens de verrouillage adaptés à mettre sous tension ledit capot rapproché de la face avant du socle ;
- le capot comporte entre ses parties d'extrémité arrière et avant une partie médiane dont la face arrière est pourvue d'une gorge destinée à épouser la tige de liaison placée dans ledit logement pour la maintenir dans celui-ci ;
- les moyens de verrouillage comprennent une vis de blocage dont le corps fileté est apte à traverser une lumière de la partie d'extrémité avant du capot pour se visser dans un puits taraudé du socle et dont la tête accessible à l'utilisateur par le dessus dudit implant est apte à se bloquer contre la face avant dudit capot ;
- ladite lumière est oblongue ;
- le corps fileté de la vis de blocage comporte une extrémité libre conique ; et
- le socle présente des faces avant et arrière à profil courbe et le capot présente un profil courbe qui suit le profil courbe de la face avant du socle.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue d'ensemble en perspective d'un implant rachidien selon l'invention ;
- la figure 2 est une vue de dessus de l'implant de la figure 1 ;
- la figure 3 est une vue en perspective de dessous de l'implant de la figure 1 ;
- la figure 4 est une vue en perspective de dessus du socle de l'implant de la figure 1 ;
- la figure 5 est une vue en perspective de dessus du capot de l'implant de la figure 1 ;
- la figure 6 est une vue en perspective du capot de la figure 5 monté en position d'ouverture sur le socle de la figure 4 recevant une tige de liaison ; et
- la figure 7 est une vue de côté du socle de la figure 4.

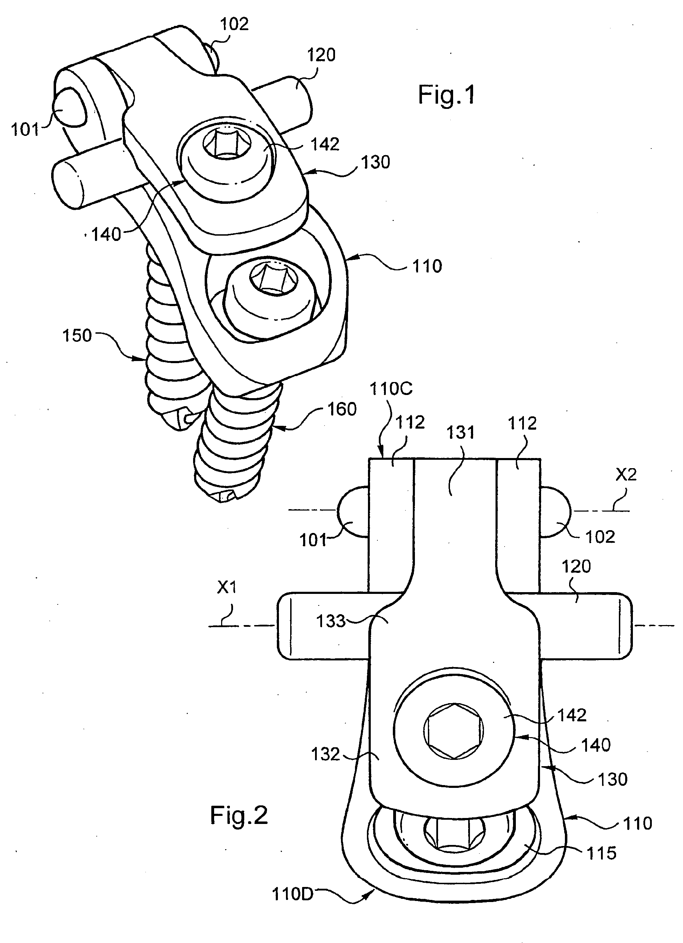

Sur les figures 1 à 3 on a représenté un implant rachidien 100 destiné à être fixé à vertèbre par voie antérieure c'est-à-dire par l'abdomen du patient.

Il permet de solidariser plusieurs vertèbres entre elles au moyen d'une tige de liaison 120 qui court le long de la colonne vertébrale, pour traiter des pathologies dégénératives ou traumatiques de la vertèbre thoracique T7 à la vertèbre lombaire L4.

Plus particulièrement l'utilisation de plusieurs de ces implants rachidiens permet de corriger de façon tridimensionnelle des déformations du rachis par une instrumentation vertébrale multi étagée.

Comme le montrent les figures, cet implant rachidien 100 comprend un socle 110 pourvu d'un logement 111 adapté à recevoir une tige de liaison 120 s'étendant de part et d'autre du socle 110.

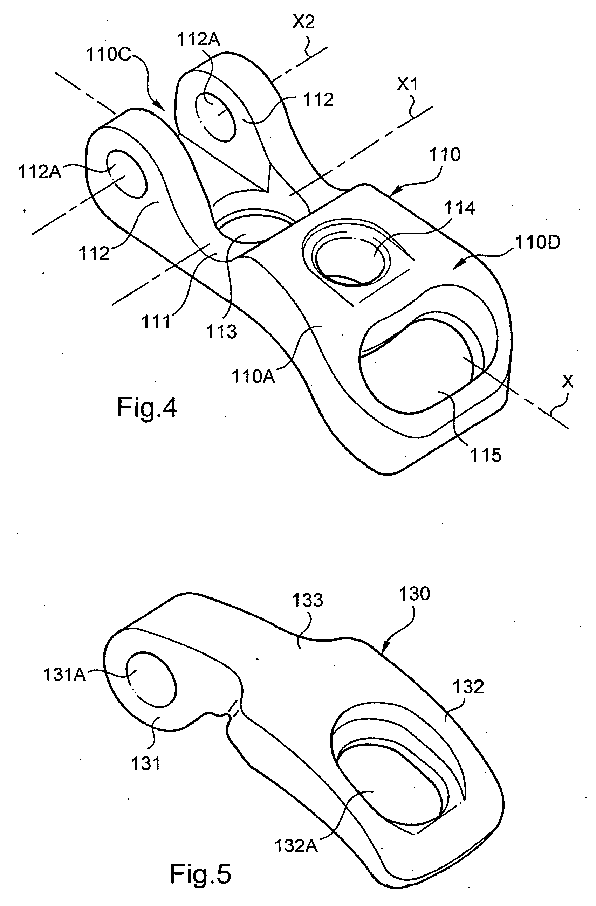

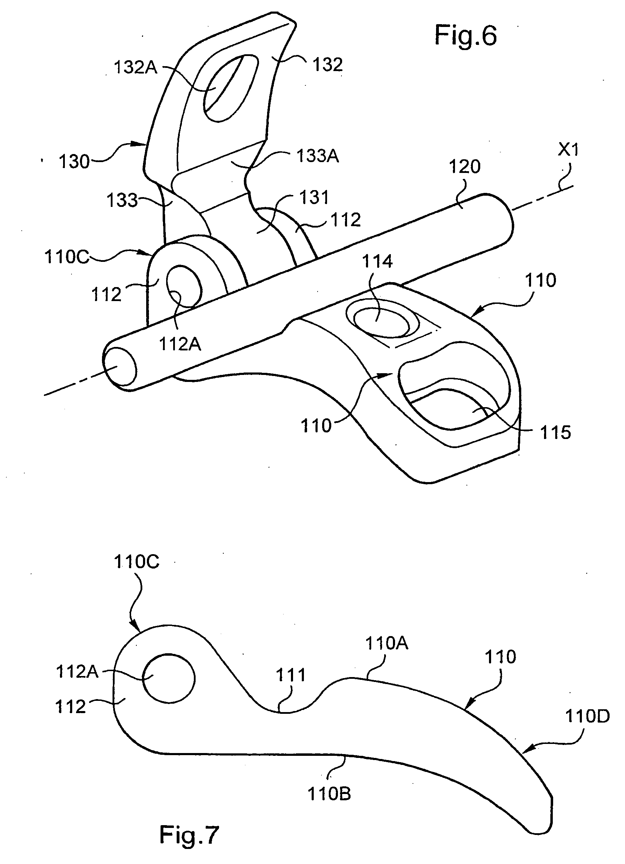

Le socle 110 s'étendant globalement selon un axe X, ledit logement 111 s'étendant selon un axe longitudinal X1 transversal à l'axe X (voir figure 4). Le logement 111 est formé par un creux de la face avant 110A du socle 110, ouvert longitudinalement et à chaque extrémité pour permettre le passage de la tige de liaison 120 (voir figures 4 et 6). Il présente un fond arrondi qui est destiné à épouser ladite tige de liaison 120 placée dans ledit logement 111 (voir figures 6 et 7).

Lorsque la tige de liaison 120 est placée dans le logement 111 elle s'étend selon l'axe longitudinal X1.

L'implant rachidien 100 comporte des moyens de blocage 130,140 de la tige de liaison 120 dans ledit logement 111.

Selon une caractéristique essentielle de cet implant, lesdits moyens de blocage comprennent un capot 130 pivotant sur le socle 110.

Plus particulièrement, comme le montrent les figures, le capot 130 est rapporté sur la partie arrière 110C du socle 110 au moyen d'un axe de pivotement autour duquel il est monté à pivotement.

Cet axe de pivotement s'étend selon l'axe X2 sensiblement parallèle à l'axe longitudinal X1 du logement 111. Il est rapporté et serré dans des ouvertures 112A d'axe X2 prévues dans des oreilles 112 parallèles du socle 110, situées en arrière du logement 111, et s'élevant perpendiculairement à la face avant 110A du socle 110.

Avantageusement, l'axe de pivotement du capot 130 comporte deux extrémités libres 101,102 de forme sphérique qui dépassent dudit socle 110. La forme sphérique desdites extrémités libres 101,102 permet de ne pas agresser les parties molles autour de la vertèbre sur laquelle est posé l'implant rachidien 100.

Comme montrent les figures 1 à 3, le capot 130 s'étend globalement transversalement audit logement 111 en le recouvrant. Il présente, de part et d'autre dudit logement 111, d'une part, une partie d'extrémité arrière 131, engagée entre les deux oreilles 112 du socle 110, pourvue d'un conduit traversant 131A, ouvert à chaque extrémité, dans lequel est enfilé l'axe de pivotement, et, d'autre part, une partie d'extrémité avant 132 agencée pour coopérer avec des moyens de verrouillage 140 adaptés à mettre sous tension ledit capot 130 rapproché de la face avant 110A du socle 110.

Le capot 130 comporte entre ses parties d'extrémité arrière et avant 131,132 une partie médiane 133 dont la face arrière comporte en creux une gorge 133A destinée à épouser la tige de liaison 120 placée dans ledit logement 111 pour la maintenir dans celui-ci.

Ici, les moyens de verrouillage comprennent une vis de bocage 140 dont le corps fileté 141 est apte à traverser une lumière 132A de la partie d'extrémité avant 132 du capot 130 pour se visser dans un puits taraudé 114 du socle 110 et dont la tête 142 accessible à l'utilisateur par le dessus dudit implant est apte à se bloquer contre la face avant dudit capot 130 (voir figures 1,2 et 3).

Préférentiellement, le corps fileté 141 de la vis de blocage 140 comporte une extrémité libre 141A conique qui dépasse de la face arrière 110B du socle 110 afin de faciliter éventuellement la pénétration de cette extrémité libre 141A dans la corticale du corps de la vertèbre.

Selon le mode de réalisation préférentiel du capot 130 représenté sur la figure 5, ladite lumière 132A est oblongue ce qui donne au chirurgien une certaine souplesse de mise en place de ladite vis de blocage 140.

Avantageusement, le socle 110 présente des faces avant et arrière 110A,110B à profil courbe et le capot 130 présente un profil courbe qui suit le profil courbe de la face avant 110A du socle 110. Par exemple la face avant 110A du socle 110 présente un rayon de courbure égal à 18 mm, la face arrière 110B de ce dernier présente à l'avant une partie courbe dont le rayon de courbure est égal à 20 mm (voir la figure 7) et la face arrière du capot 130 présente un rayon de courbure égal à 20 mm. Ainsi, l'implant rachidien 100 en position sur la vertèbre présente un encombrement en hauteur réduit, inférieur ou égal à 10 mm. Cette configuration courbe de l'implant permet de protéger les vaisseaux sanguins adjacents à la vertèbre.

Par ailleurs, le socle 110 comporte, d'une part, en regard dudit logement 111 une première ouverture traversante 113 qui débouche du côté de la face avant 110A du socle 110 en retrait du fond dudit logement 111, et, d'autre part, à son extrémité avant 110D une deuxième ouverture traversante 115, lesdites première et deuxième ouvertures traversantes 113,115 présentant des axes Y1,Y2 convergents pour la mise en place de deux vis 150,160 de fixation du socle 110 à une vertèbre.

L'ouverture traversante 115 prévue à l'avant du socle 110 présente une forme oblongue ce qui donne au chirurgien une souplesse de mise en place de la vis 160 dans la vertèbre en fonction de la position de l'autre vis 150.

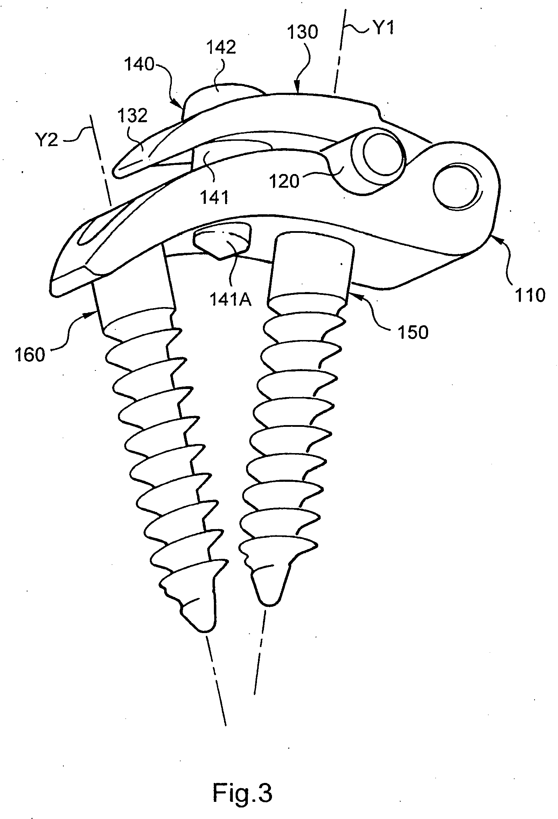

Comme le montrent plus particulièrement les figures 2 et 3, avantageusement, le capot 130 en position de fermeture sur le socle présente une partie d'extrémité avant 132 qui s'étend au-dessus d'une partie de la deuxième ouverture traversante 115 pour former une butée anti-recul de la vis 160 introduite dans ladite deuxième ouverture traversante 115. Toutefois, avantageusement, le recouvrement de ladite ouverture traversante 115 par le capot 130 est tel qu'il permet encore au chirurgien d'accéder avec un tournevis à la tête de la vis 160 introduite dans la vertèbre via la deuxième ouverture traversante 115 pour la serrer sur le socle 110.

Chacune des pièces de l'implant rachidien 100 est réalisée en titane ou alliage de titane biocompatible.

La mise en place de cet implant sur une vertèbre est réalisée de la manière suivante.

Tout d'abord le socle 110 avec le capot 130 ouvert est positionné sur la partie antéro-latérale du corps vertébral.

Puis la vis 150 postérieure est vissée au travers de l'ouverture traversante 113 dans l'os cortical de la vertèbre sans rechercher forcément une prise bi-corticale. Cette vis 150 postérieure est serrée sur le socle 110 pour fixer l'implant rachidien 100 à la vertèbre.

Le chirurgien visse ensuite la vis 160 antérieure au travers de l'ouverture traversante 115 dans l'os cortical de la vertèbre de façon convergente à la vis 150 postérieure. Toutefois, cette vis 160 antérieure n'est pas serrée immédiatement sur le socle 110 pour permettre ultérieurement au chirurgien de légèrement bouger ledit implant rachidien 100 afin d'ajuster sa position finale sur la vertèbre.

La tige de liaison 120 est descendue dans le fond du logement 111 du socle 110 de sorte qu'elle recouvre la tête de la vis 150 postérieure en appui dans le fond de l'ouverture traversante 113.

Le capot 130 est alors rabattu par pivotement sur le socle 110 et la vis de blocage 140 est introduite dans le puits taraudé 114 du socle 110 au travers de la lumière 132A de manière que le capot 130 maintient ladite tige de liaison 120 en position dans le logement 111 tout en autorisant sa rotation et/ou sa translation dans ledit logement 111.

Le chirurgien pose alors de la même manière les autres implants rachidiens du même type sur toutes les vertèbres à instrumentaliser puis il ajuste la position de l'implant rachidien 100 sur la vertèbre en fonction du cintrage de la tige de liaison 120 qui passe dans les logements 111 desdits implants.

Enfin le chirurgien serre la vis 160 antérieure sur le socle 110 de l'implant rachidien 100 puis il serre la vis de blocage 140 dans le puits taraudé 114 de manière à bloquer la tige de liaison 120 dans le logement 111 et à verrouiller ainsi l'implant rachidien 100 sur celle-ci.

L'implant rachidien 100 selon l'invention présente les avantages suivants :
- le capot pivotant permet de positionner aisément la tige de liaison dans le logement du socle et procure un maintien de cette tige tout en autorisant sa rotation et/ou sa translation avant le blocage final ;
- l'implant rachidien présente une configuration courbe à faible hauteur qui permet de protéger les vaisseaux sanguins avoisinant la vertèbre,
- le capot et la tige de liaison placée dans le logement du socle forment un système de butée anti-recul pour les vis de fixation à la vertèbre ;
- l'implant rachidien est agencé de manière à permettre au chirurgien de réaliser toutes les actions de fixation des vis par le dessus de l'implant ;
- l'implant se présente en une seule taille valable pour le côté droit ou gauche et adaptée à différentes tailles de vertèbres. Ainsi il peut être posé sur un rachis d'adulte ou d'enfant.

## Revendications

1. Implant rachidien (100) comprenant :
- un socle (110) pourvu d'un logement (111) adapté à recevoir une tige de liaison (120) s'étendant de part et d'autre du socle (110), et comportant, d'une part, en regard dudit logement (111) une première ouverture traversante (113) qui débouche du côté de la face avant (110A) du socle (110) en retrait du fond dudit logement (111), et, d'autre part, à son extrémité avant une deuxième ouverture traversante (115), lesdites première et deuxième ouvertures traversantes (113,115) présentant des axes (Y1,Y2) convergents pour la mise en place de deux vis (150,160) de fixation du socle (110) à une vertèbre, et
- des moyens de blocage (130,140) de la tige de liaison (120) dans ledit logement (111) comprenant un capot (130) pivotant sur le socle (110),
**caractérisé en ce que** le capot (130) en position de fermeture sur le socle présente une partie d'extrémité avant (132) qui s'étend au-dessus d'une partie de la deuxième ouverture traversante (115) pour former une butée anti-recul de la vis (160) introduite dans ladite deuxième ouverture traversante (115).

2. Implant rachidien selon la revendication 1, **caractérisé en ce que** ledit capot (130) est monté à pivotement autour d'un axe de pivotement qui s'étend sensiblement parallèlement à un axe longitudinal (X1) du logement (111) selon lequel est destinée à s'étendre ladite tige de liaison (120).

3. Implant rachidien selon la revendication 2, **caractérisé en ce que** ledit axe de pivotement est rapporté et serré dans des ouvertures (112A) du socle (110).

4. Implant rachidien selon l'une des revendications 2 ou 3, **caractérisé en ce que** l'axe de pivotement comporte deux extrémités libres (101,102) de forme sphérique qui dépassent dudit socle (110).

5. Implant rachidien selon l'une des revendications précédentes, **caractérisé en ce que** le capot (130) s'étend globalement transversalement audit logement (111) en le recouvrant et il présente de part et d'autre dudit logement (111) une partie d'extrémité arrière (131) pourvue d'un conduit (131A) ouvert à chaque extrémité pour la mise en place d'un axe de pivotement et une partie d'extrémité avant (132) agencée pour coopérer avec des moyens de verrouillage (140) adaptés à mettre sous tension ledit capot (130) rapproché de la face avant (110A) du socle (110).

6. Implant rachidien selon la revendication 5, **caractérisé en ce que** le capot (130) comporte entre ses parties d'extrémité arrière et avant (131,132) une partie médiane (133) dont la face arrière est pourvue d'une gorge (133A) destinée à épouser la tige de liaison (120) placée dans ledit logement (111) pour la maintenir dans celui-ci.

7. Implant rachidien selon l'une des revendications 5 et 6, **caractérisé en ce que** la partie d'extrémité avant du (132) capot (130) comporte une lumière (132A) et le socle (110) comporte un puits taraudé (114), ledit implant rachidien comprenant des moyens de verrouillage comprenant une vis de blocage (140) dont le corps fileté (141) est apte à traverser la lumière (132A) de la partie d'extrémité avant (132) du capot (130) pour se visser dans le puits taraudé (114) du socle (110) et dont la tête (142) accessible à l'utilisateur par le dessus dudit implant est apte à se bloquer contre la face avant dudit capot (130).

8. Implant rachidien selon la revendication 7, **caractérisé en ce que** ladite lumière (132A) est oblongue.

9. Implant rachidien selon l'une des revendications 7 ou 8, **caractérisé en ce que** le corps fileté (141) de la vis de blocage (140) comporte une extrémité libre (141A) conique.

10. Implant rachidien selon l'une des revendications précédentes, **caractérisé en ce que** le socle (110) présente des faces avant et arrière (110A,110B) à profil courbe et le capot (130) présente un profil courbe qui suit le profil courbe de la face avant (110A) du socle (110).

## Claims

1. A spinal implant (100) comprising:
a base (110) provided with a housing (111) adapted to receive a connecting rod (120) extending across the base (110) from side to side, and including firstly, facing said housing (111), a first through opening (113) that opens out to the front face (110A) of the base (110) and that is set back from the bottom of said housing (111), and secondly at its front-end, a second through opening (115), said first and second through openings (113, 115) presenting converging axes (Y1, Y2) for installing two fastener screws (150, 160) for fastening the base (110) to a vertebra; and
blocking means (130, 140) for blocking the connecting rod (120) in said housing (111), the blocking means comprising a cover (130) pivotally mounted on the base (110);
the spinal implant being **characterized in that** the cover (130) in its closed position on the base presents a front end portion (132) that extends over a portion of the second through opening (115) to form a backstop for the screw (160) inserted in said second through opening (115).

2. A spinal implant according to claim 1, **characterized in that** said cover (130) is mounted to pivot about a pivot pin that extends substantially parallel to a longitudinal axis (X1) of the housing (111) and along which said connecting rod (120) is to extend.

3. A spinal implant according to claim 2, **characterized in that** said pivot pin is fitted and clamped in openings (112A) of the base (110).

4. A spinal implant according to claim 2 or claim 3, **characterized in that** the pivot pin includes two free ends (101, 102) of spherical shape that project beyond said base (110).

5. A spinal implant according to any preceding claim, **characterized in that** the cover (130) extends generally transversely relative to said housing (111), covering the housing, and presenting on either side of said housing (111) a rear end portion (131) provided with a bore (131A) that is open at each end for installing a pivot pin; and a front end portion (132) arranged to co-operate with locking means (140) adapted to put said cover (130) under tension close to the front face (110A) of the base (110).

6. A spinal implant according to claim 5, **characterized in that** the cover (130) includes, between its rear and front end portions (131, 132), a middle portion (133) having its rear face provided with a groove (133A) designed to fit closely against the connecting rod (120) placed in said housing (111) in order to hold it therein.

7. A spinal implant according to claim 5 or claim 6, **characterized in that** the front end portion (132) of the cover (130) includes a slot (132A) and the base (110) includes a tapped well (114), said spinal implant including locking means comprising a blocking screw (140) with a threaded shank (141) suitable for passing through the slot (132A) of the front end portion (132) of the cover (130) to be screwed into the tapped well (114) of the base (110), and having a head (142) that is accessible to the user from above said implant and that is suitable for becoming blocked against the front face of said cover (130).

8. A spinal implant according to claim 7, **characterized in that** said slot (132A) is oblong.

9. A spinal implant according to claim 7 or claim 8, **characterized in that** the threaded shank (141) of the blocking screw (140) includes a conical free end (141A).

10. A spinal implant according to any preceding claim, **characterized in that** the base (110) presents front and rear faces (110A, 110B) of curved profile, and the cover (130) presents a curved profile that follows the curved profile of the front face (110A) of the base (110).

## Patentansprüche

1. Rückenmarksimplantat (100) mit:
- einem Sockel (110), der mit einer Aufnahme (111) versehen ist, die einen Verbindungsstift (120) aufnehmen kann, der sich auf beiden Seiten des Sockels (110) erstreckt, wobei der Sockel zum einen gegenüber dieser Aufnahme (111) eine erste Durchgangsöffnung (113) umfasst, die auf der Vorderseite (110A) des Sockels (110), in Bezug zum Boden dieser Aufnahme (111) zurückgesetzt, mündet, und zum anderen an seinem vorderen Ende eine zweite Durchgangsöffnung (115), wobei die erste und zweite Durchgangsöffnung (113, 115) für das Anbringen von zwei Befestigungsschrauben (150, 160) des Sockels (110) an einen Wirbelkörper zusammenlaufende Achsen (Y1, Y2) aufweisen, und
- Mitteln zum Blockieren (130,140) des Verbindungsstifts (120) in der Aufnahme (111), die eine auf dem Sockel (110) schwenkbare Abdeckung (130) umfassen,
**dadurch gekennzeichnet, dass** die Abdeckung (130) in der Schließposition auf dem Sockel einen vorderen Endbereich (132) aufweist, der sich über einen Teil der zweiten Durchgangsöffnung (115) erstreckt, um einen Anschlag gegen das Zurückschieben der in die zweite Durchgangsöffnung (115) gesteckte Schraube (160) zu bilden.

2. Rückenmarksimplantat nach Anspruch 1, **dadurch gekennzeichnet, dass** die Abdeckung (130) um eine Schwenkachse schwenkbar montiert ist, die sich im Wesentlichen parallel zu einer Langsachse (X1) der Aufnahme (111) erstreckt, entlang der sich der Verbindungsstift (120) erstrecken soll.

3. Rückenmarksimplantat nach Anspruch 2, **dadurch gekennzeichnet, dass** die Schwenkachse in Öffnungen (112A) des Sockels (110) eingeschoben und angezogen wird.

4. Rückenmarksimplantat nach einem der Ansprüche 2 oder 3, **dadurch gekennzeichnet, dass** die Schwenkachse zwei kugelförmige freie Enden (101, 102) umfasst, die über den Sockel (110) hinausstehen.

5. Rückenmarksimplantat nach einem der vorausgehenden Ansprüche, **dadurch gekennzeichnet, dass** sich die Abdeckung (130) im Allgemeinen quer zur Aufnahme (111) erstreckt und diese abdeckt und auf beiden Seiten der Aufnahme (111) einen hinteren Endbereich (131) mit einer Durchführung (131A) aufweist, die an jedem Ende für das Anbringen einer Schwenkachse offen ist, sowie einen vorderen Endbereich (132), der zum Zusammenwirken mit den Verriegelungsmitteln (140) angebracht ist die die der Vorderseite (110A) des Sockels (110) angenäherte Abdeckung (130) spannen können.

6. Rückenmarksimplantat nach Anspruch 5, **dadurch gekennzeichnet, dass** die Abdeckung (130) zwischen ihrem vorderen und hinteren Endbereich (131, 132) einen Mittelbereich (133) umfasst, dessen Rückseite mit einer Vertiefung (133A) versehen ist, die sich dem in die Aufnahme (111) gesteckten Verbindungsstift (120) anpassen soll, um diesen in derAufnahme zu halten.

7. Ruckenmarksimplantat nach einem der Ansprüche 5 und 6, **dadurch gekennzeichnet, dass** der vordere Endbereich (132) der Abdeckung (130) eine Öffnung (132A) aufweist und der Sockel (110) eine Gewindebohrung (114) umfasst, wobei das Rückenmarksimplantat Verriegelungsmittel mit einer Klemmschraube (140) umfasst, deren Gewindebolzen (141) die Öffnung (132A) des vorderen Endbereichs (132) der Abdeckung (130) durchdringen kann, um sich in die Gewindebohrung (114) des Sockels (110) drehen zu lassen und deren für den Anwender über die Oberseite des Implantats zugänglicher Kopf (142) an der Vorderseite der Abdeckung (130) arretiert werden kann.

8. Rückenmarksimplantat nach Anspruch 7, **dadurch gekennzeichnet, dass** die Öffnung (132A) länglich ist.

9. Rückenmarksimplantat nach einem der Ansprüche 7 oder 8, **dadurch gekennzeichnet, dass** der Gewindebolzen (141) der Klemmschraube (140) ein kegelförmiges freies Ende (141A) umfasst.

10. Rückenmarksimplantat nach einem der vorausgehenden Ansprüche, **dadurch gekennzeichnet, dass** der Sockel (110) Vorder und Rückseiten (110A, 110B) mit einem gekrümmten Profil aufweist und die Abdeckung (130) ein gekrümmtes Profil aufweist, das dem gekrümmten Profil der Vorderseite (110A) des Sockels (110) folgt.
